(19) **Europäisches Patentamt**

**European Patent Office**

**Office européen des brevets**

(11) Veröffentlichungsnummer: **0 238 966 B1**

(12) **EUROPÄISCHE PATENTSCHRIFT**

(45) Veröffentlichungstag der Patentschrift: **15.01.92**

(51) Int. Cl.⁵: **A61N 1/368**

(21) Anmeldenummer: **87103786.7**

(22) Anmeldetag: **16.03.87**

(54) **Vorhofsteuerbarer Herzschrittmacher.**

(30) Priorität: **18.03.86 DE 3609072**

(43) Veröffentlichungstag der Anmeldung:
**30.09.87 Patentblatt 87/40**

(45) Bekanntmachung des Hinweises auf die
Patenterteilung:
**15.01.92 Patentblatt 92/03**

(84) Benannte Vertragsstaaten:
**DE FR GB IT NL SE**

(56) Entgegenhaltungen:
**EP-A- 0 063 097**
**EP-A- 0 076 363**
**EP-A- 0 147 820**
**DE-A- 2 712 617**
**US-A- 4 378 020**

(73) Patentinhaber: **Siemens Elema AB**
**Röntgenvägen 2**
**S-171 95 Solna 1(SE)**

(84) Benannte Vertragsstaaten:
**SE**

Patentinhaber: **SIEMENS AKTIENGESELL-**
**SCHAFT**
**Wittelsbacherplatz 2**
**W-8000 München 2(DE)**

(84) Benannte Vertragsstaaten:
**DE FR GB IT NL**

(72) Erfinder: **Lindgren, Anders**
**Sportvaegen 24**
**S-18 340 Taeby(SE)**

**Beschreibung**

Die Erfindung betrifft einen vorhofsteuerbaren Herzschrittmacher gemäss dem Oberbegriff des Anspruches 1. Bei derartigen Herzschrittmachern erfolgt im vorhofgesteuerten Betrieb die Erzeugung der ventrikulären Stimulierungsimpulse nach einer Verzögerungszeit AV nach dem Auftreten eines Vorhofsignales. In Abhängigkeit von dem vorgegebenen kleinsten synchronen Intervall (SSI) kann die Stimulationsfrequenz innerhalb eines relativ weiten Bereiches von z.B. 50 bis 150 Herzschlägen pro Minute variieren. Derartige Herzschrittmacher enthalten eine Grenzstufe, die die höchste Impulsrate festlegt. Tritt ein Vorhofsignal vor einer vorgegebenen Grenze, die sich aus der Differenz zwischen dem kleinsten synchronen Intervall und der AV-Verzögerung ergibt, auf, schaltet der Herzschrittmacher auf den ventrikelinhibierten Betrieb um.

Wie sich schon frühzeitig beim Einsatz vorhofgesteuerter Herzschrittmacher gezeigt hat, treten neben der verbesserten Hämodynamik auch eine Reihe von Nachteilen auf. So kann eine retrograde Überleitung im Herzen zu einer herzschrittmacherinduzierten Tachykardie (PMT, Pacemaker-Mediated Tachycardia) führen. Um das zu verhindern, wurde bereits vorgeschlagen, die Refraktärzeit im Vorhof zu verlängern, was aber eine Herabsetzung der oberen Herzschlagfrequenz nach sich zieht. Ein anderer Vorschlag läuft darauf hinaus, die Refraktärzeit im Vorhof nur dann zu verlängern, wenn die Wahrscheinlichkeit für eine retrograde VA-Überleitung hoch ist, wie beispielsweise nach einer vorzeitigen Ventrikelkontraktion (PVC). Wie neuere Untersuchungen gezeigt haben, kann aber diese Massnahme in besonderen Situationen zu einer totalen Inhibierung des Herzschrittmachers führen (Zeitschrift Pace, Vol 8, Nov/Dez. 1985).

Immer wenn der Herzschrittmacher aus dem ventrikelinhibierten Betrieb wieder auf vorhofgesteuerten Betrieb umschalten soll, ist das Risiko besonders gross, dass die Synchronisierung gerade bei einer durch retrograde Überleitung ausgelösten Vorhofaktivität startet und damit eine herzschrittmacherinduzierte Tachykardie einleitet.

Der vorliegenden Erfindung liegt die Aufgabe zugrunde, bei einem Herzschrittmacher der eingangs genannten Art beim Übergang zum vorhofgesteuerten Betrieb das Auftreten einer herzschrittmacherinduzierten Tachykardie einfach und sicher zu verhindern, ohne andere Herzschrittmachereigenschaften wie z.B. die höchste mögliche Herzschlagfrequenz nachteilig zu beeinflussen.

Diese Aufgabe wird erfindungsgemäss durch die Merkmale des Anspruchs 1 gelöst. Für den Fall, dass erst bei dem 2. oder einem späteren Vorhofsignal nach dem ventrikulären Ereignis zum vorhofgesteuerten Betrieb übergegangen wird, löst das 1. Vorhofsignal noch keine Stimulierung im Ventrikel aus. Das 2. Vorhofsignal kann daher nicht durch retrograde Überleitung eines Ventrikelsignales hervorgerufen sein, sondern ist eine echte Vorhofaktivität.

Wenn eine derartige echte Vorhofaktivität nicht auftritt, ist vorgesehen, dass nach dem 1. Vorhofsignal erst nach einer Verzögerungszeit Δ t zu vorhofgesteuertem Betrieb übergegangen wird. Gemäss einer Weiterbildung kann zunächst eine AV-sequentielle Stimulierung des Herzens erfolgen, um das Reizleitungssystem in einem antigraden Weg zu depolarisieren und dadurch das Entstehen einer herzschrittmacherinduzierten Tachykar die zu verhindern. Die Verzögerungszeit muss dabei so gross gewählt sein, dass sich der Vorhof nach dem 1. Vorhofsignal repolarisiert hat und dadurch überhaupt bei der AV-sequentiellen Stimulierung depolarisierbar ist.

In einer vorteilhaften Weiterbildung der Erfindung ist vorgesehen, dass die Verzögerungszeit programmierbar ist. Erfahrungsgemäss sollte sie mindestens 200 ms betragen.

In Weiterbildung des Herzschrittmachers ist vorgesehen, dass eine Schaltstufe den Herzschrittmacher auf VVI-Betrieb umschaltet, wenn das Vorhofsignal vor einer vorgebbaren zeitlichen Grenze auftritt. Automatisch wird erneut auf vorhofgesteuerten Betrieb zurückgeschaltet, wenn das Vorhofsignal wieder nach der Grenze auftritt. Auch hier erfolgt das Zurückschalten mit der notwendigen Verzögerung, um sicher zu vermeiden, dass eine Tachykardie induziert wird.

Die Erfindung ist nachfolgend anhand eines in der Zeichnung dargestellten Ausführungsbeispieles näher erläutert. Es zeigen:

Fig. 1    Impulsverläufe zur Erläuterung des Erfindungsgedankens,

Fig. 2    einen Herzschrittmacher nach der Erfindung.

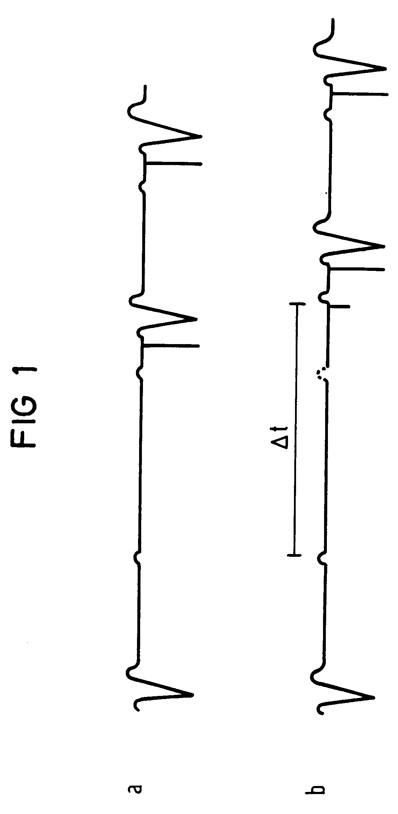

In Fig. 1a ist ein erster möglicher Übergang zum vorhofgesteuerten Betrieb dargestellt. Nach dem den Übergang einleitenden QRS-Signal wird durch die erste P-Welle kein synchroner Ventrikel-Stimulierungsimpuls ausgelöst. Diese erste P-Welle wird praktisch unterdrückt. Erst bei der zweiten P-Welle beginnt die synchrone Vorhofsteuerung. Kann die erste P-Welle noch durch retrograde Überleitung des QRS-Signales hervorgerufen sein, so ist das für die zweite P-Welle unmöglich. Hierbei handelt es sich sicher um eine echte Atriumaktivität.

Die Fig. 1b zeigt einen zweiten möglichen Übergang zum vorhofgesteuerten Betrieb. Wiederum wird der Übergang durch ein QRS-Signal eingeleitet. Das erste diesem Signal folgende Vorhofsignal löst eine Verzögerungszeit Δt aus. Diese muss so gross gewählt sein, dass sich der Vorhof nach dem ersten Vorhofsignal repolarisiert hat. Am Ende dieser Verzögerungszeit Δt erfolgt eine AV-sequentielle Stimulierung, die das Reizleitungssystem in einem antigraden Weg zunächst depolarisiert.

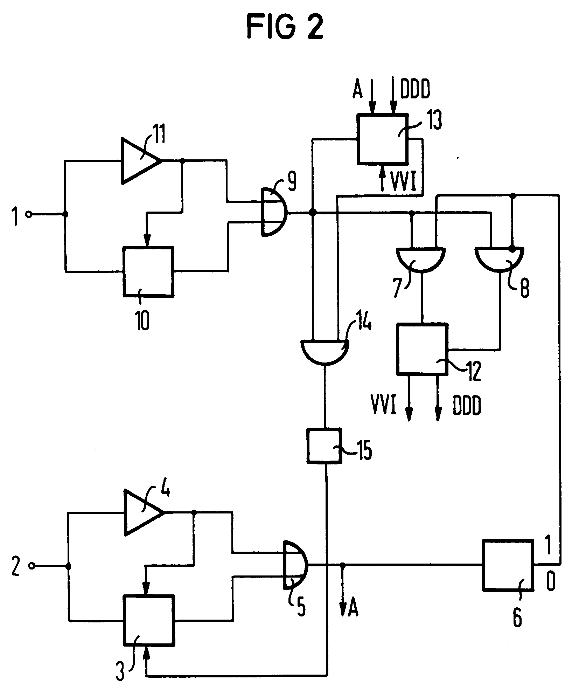

Im Schaltbild gemäss Fig. 2 werden die Vorhofsignale einer Klemme 1 zugeführt und die Ventrikelsignale liegen an einer Klemme 2. Die Impulse einer ventrikulären Ausgangsstufe 3 und eines ventrikulären Detektors 4 gehen über ein ODER-Gatter 5 und starten eine astabile Kippstufe 6. Die Schaltzeit dieser astabilen Kippstufe entspricht der Differenz zwischen dem kleinsten synchronen Intervall und der eingestellten AV-Verzögerungszeit. Während des astabilen Zustandes liegt am Ausgang der Kippstufe eine logische EINS, ansonsten eine logische NULL an. Der Ausgang der Kippstufe 6 ist auf ein UND-Gatter 7 und gleichzeitig auf den invertierten Eingang eines UND-Gatters 8 geschaltet. Auf die anderen Eingänge der beiden UND-Gatter ist der Ausgang eines ODER-Gatters 9 geschaltet, das seine Eingangssignale von der Ausgangsstufe 10 bzw. dem Detektor 11 für die Atriumaktivität (P-Welle) erhält.

Wenn nach einem ventrikulären Ereignis in der Zeit, in der das Ausgangssignal der astabilen Kippstufe 6 einer logischen EINS entspricht, eine Atriumaktivität detektiert wird, d.h. wenn die abgefühlten Vorhofaktivitäten die höchste synchrone Frequenz des Herzschrittmachers überschreiten, wird die Mode-Kontroll Logik 12 durch das Ausgangssignal des UND-Gatters 7 zu einem Mode-Wechsel vom DDD-Betrieb zum VVI-Betrieb veranlasst. Wird nachfolgend eine Vorhofaktivität erst dann detektiert, wenn das Ausgangssignal der Kippstufe 6 bereits wieder auf NULL zurückgegangen ist, so wird über das Ausgangssignal des UND-Gatters 8 die Mode-Kontroll-Logik 12 dazu veranlasst, den Herzschrittmacher wieder auf DDD-Betrieb umzuschalten.

Das Ausgangssignal des ODER-Gatters 9 ist weiterhin auf einen P-Wellenzähler 13 geschaltet, dessen Ausgangssignal auf ein weiteres UND-Gatter 14 gegeben ist. Auf den zweiten Eingang dieses UND-Gatters ist der Ausgang des ODER-Gatters 9 geschaltet. Der Ausgang des UND-Gatters 14 steuert ein AV-Verzögerungsglied 15 an, dessen Ausgangssignal die ventrikuläre Ausgangsstufe 3 ansteuert. Der P-Wellenzähler 13 erhält weiterhin ein Steuersignal A entsprechend einem ventrikulären Ereignis sowie die jeweiligen Ausgangssignale der Mode-Kontroll-Logik 12. Für die VVI-Mode wird der Zähler 13 jeweils auf Null zurückgesetzt. Beim Vorliegen des erneuten DDD-Signales der Mode-Kontroll-Logik wird der Zähler durch das erste Signal A entsprechen einem ventrikulären Ereignis freigegeben. Beim Zählerwert zwei und höher liegt am Ausgang des Zählers 13 eine logische EINS an. In diesem Fall werden die Ausgangssignale des ODER-Gatters 9 über das UND-Gatter 14 und die AV-Verzögerungsstufe 15 auf die ventrikuläre Ausgangsstufe 3 durchgeschaltet. Das erste Vorhofsignal nach dem Umschalten auf DDD-Mode wird jedoch unterdrückt, da der Zähler beim Zählwert 1 am Ausgang noch eine logische NULL aufweist und das UND-Gatter 14 damit gesperrt ist.

Mit Hilfe dieser Schaltung wird erreicht, das jedesmal, wenn die Mode-Kontroll-Logik den Herzschrittmacher von VVI auf DDD-Betrieb umschaltet, dieser Übergang erst beim zweiten Vorhofsignal erfolgt, das dem ventrikulären Ereignis folgt, welches die Umschaltung ausgelöst hat. Ebenso ist es möglich, dass der Übergang erst bei einem späteren Vorhofsignal erfolgt. Vorteilhafterweise sollte das im Zähler 13 programmierbar sein.

Beispielhaft wurde hier in einem Blockschaltbild die Prinzipschaltung eines Herzschrittmachers nach der Erfindung gewählt, bei dem der Herzschrittmacher zwischen VVI- und DDD-Moden wechseln kann. Im Rahmen der vorliegenden Erfindung sind auch Wechsel zwischen anderen Moden möglich, wichtig ist jeweils nur der Übergang zum vorhofsynchronen Betrieb.

Bezugszeichenliste

| | |
|---|---|
| 1,2 = | Klemme |
| 3,10 = | Ausgangsstufe |
| 4,11 = | Detektor |
| 5,9 = | ODER-Gatter |
| 6 = | Kippstufe |
| 7,8,14 = | UND-Gatter |
| 12 = | Mode-Kontroll-Logik |
| 13 = | Zähler |
| 15 = | AV-Verzögerungsglied |

**Patentansprüche**

1. Vorhofsteuerbarer Herzschrittmacher mit einer Schaltstufe zum Umschalten des Herzschrittmachers zwischen verschiedenen Moden darunter einem vorhofgesteuerten Betrieb, **dadurch gekennzeichnet,** daß beim Umschalten auf den vorhofgesteuerten Betrieb die Schaltstufe diese Umschaltung erst nach einer Verzögerung nach dem letzten ventrikulären Ereignis (Ventrikelstimulationsimpuls oder QRS) ausführt und daß die Verzögerung so lange gewählt ist, daß ein erstes Vorhofsignal nach dem letzten ventrikulären Ereignis vor Ablauf der Verzögerung auftritt und für den vorhofgesteuerten Betrieb unwirksam ist.

2. Herzschrittmacher nach Anspruch 1, **dadurch gekennzeichnet,** dass die Schaltstufe diese Umschaltung erst beim 2. oder einem späteren Vorhofsignal nach dem letzten ventrikulären Ereignis ausführt.

3. Herzschrittmacher nach Anspruch 2, **dadurch gekennzeichnet,** dass die schaltstufe einen P-Wellenzähler (13) aufweist, der beim Umschalten durch das ventrikuläre Ereignis freigegeben und gegebenenfalls auf einen vorgebbaren Ausgangswert zurückgestellt wird.

4. Herzschrittmacher nach Anspruch 1, **dadurch gekennzeichnet,** dass die Umschaltung nach einer vorgebbaren Verzögerungszeit Δt nach dem 1. Vorhofsignal nach dem letzten ventrikulären Ereignis erfolgt.

5. Herzschrittmacher nach Anspruch 4, **dadurch gekennzeichnet,** dass die Verzögerungszeit Δt programmierbar ist.

6. Herzschrittmacher nach Anspruch 4 oder 5, **dadurch gekennzeichnet,** dass die Verzögerungszeit Δt mindestens 200 ms beträgt.

7. Herzschrittmacher nach einem der Ansprüche 4 bis 6, **dadurch gekennzeichnet,** dass nach dem Umschalten zunächst eine AV-sequentielle Stimulierung erfolgt.

8. Herzschrittmacher nach einem der Ansprüche 1 bis 7 mit Abhängigkeit vom Auftreten von Vorhofsignalen gesteuerter Schaltstufe, die den Schrittmacher beim Auftreten mindestens eines Vorhofsignales vor einer vorgegebenen Grenze, die sich aus der Differenz zwischen dem kleinsten synchronen Intervall (SSI) und der AV-Verzögerung ergibt, auf ventrikelinhibierten Betrieb (VVI-Betrieb) umschaltet, **dadurch gekennzeichnet,** dass die Umschaltung auf vorhofgesteuerten Betrieb beim nachfolgenden Auftreten der Vorhofsignale nach der Grenze erfolgt.

## Claims

1. Cardiac pacemaker controlled by the atrium, with a switching stage for switching the cardiac pacemaker between different modes, among them an atrium-controlled mode, characterised in that, upon switching to the atrium-controlled mode, the switching stage effects this switch only after a delay following the most recent ventricular event (ventricular stimulation pulse or QRS), and in that the delay is selected of such a length that a first atrial signal following the most recent ventricular event appears before expiry of the delay and is inactive for the atrium-controlled mode.

2. Cardiac pacemaker according to Claim 1, characterised in that the switching stage effects this switch only upon the second or a later atrial signal following the most recent ventricular event.

3. Cardiac pacemaker according to Claim 2, characterised in that the switching stage has a P-wave counter (13) which, upon switching, is released by the ventricular event and, if appropriate, is reset to a predeterminable initial value.

4. Cardiac pacemaker according to Claim 1, characterised in that the switching occurs after a predeterminable delay time Δt after the first atrial signal following the most recent ventricular event.

5. Cardiac pacemaker according to Claim 4, characterised in that the delay time Δt is programmable.

6. Cardiac pacemaker according to Claim 4 or 5, characterised in that the delay time Δt is at least 200 ms.

7. Cardiac pacemaker according to one of Claims 4 to 6, characterised in that initially an AV-sequential stimulation occurs following switching.

8. Cardiac pacemaker according to one of Claims 1 to 7, with a switching stage which is controlled as a function of the appearance of atrial signals and which switches the pacemaker to ventricle-inhibited mode (VVI mode) upon the appearance of at least one atrial signal before a predetermined limit which is obtained from the difference between the smallest synchronous interval (SSI) and the AV-delay, characterised in that the switching to atrium-controlled mode takes place upon the subsequent appearance of the atrial signals after the limit.

## Revendications

1. Stimulateur cardiaque pouvant être commandé au moyen d'une commande atriale, comportant un circuit de commutation servant à réaliser une commutation entre différents modes, incluant un fonctionnement à commande atriale, caractérisé par le fait que, lors de la commutation sur le fonctionnement à commande atriale, le circuit de commutation exécute cette commutation uniquement au bout d'un retard après la dernière action ventriculaire (impulsion de stimulation ventriculaire ou QRS) et que le retard est choisi suffisamment long pour qu'un premier signal atrial se manifestant après la dernière action ventriculaire apparaisse avant la fin du retard et soit inactif pour le fonctionnement à commande atriale.

2. Stimulateur cardiaque suivant la revendication 1, caractérisé par le fait que le circuit de commutation exécute cette commutation uniquement lors de l'apparition du second signal atrial ou d'un signal atrial ultérieur après la dernière action ventriculaire.

3. Stimulateur cardiaque suivant la revendication 2, caractérisé par le fait que le circuit de commutation comprend un compteur d'ondes P (13), qui est libéré lors de la commutation par l'action ventriculaire et est éventuellement ramené à une valeur initiale pouvant être prédéterminée.

4. Stimulateur cardiaque suivant la revendication 1, caractérisé par le fait que la commutation s'effectue après un retard $\Delta t$ pouvant être prédéterminé, après le 1-er signal atrial, après la dernière action ventriculaire.

5. Stimulateur cardiaque suivant la revendication 4, caractérisé par le fait que le retard $\Delta t$ est programmable.

6. Stimulateur cardiaque suivant la revendication 4 ou 5, caractérisé par le fait que le retard $\Delta t$ est égal à au moins 200 ms.

7. Stimulateur cardiaque suivant l'une des revendications 4 à 6, caractérisé par le fait qu'après la commutation intervient tout d'abord une stimulation séquentielle AV.

8. Stimulateur cardiaque suivant l'une des revendications 1 à 7 comportant un circuit de commutation, qui est commandé en fonction de l'apparition de signaux atriaux qui commutent le stimulateur sur le fonctionnement à inhibition ventriculaire (fonctionnement VVI) lors de l'apparition d'au moins un signal atrial avant une limite prédéterminée qui est fournie par la différence entre l'intervalle synchrone le plus faible (SSI) et le retard AV, caractérisé par le fait que la commutation sur le fonctionnement à commande atriale s'effectue lors de l'apparition ultérieure des signaux atriaux après la limite.

FIG 1

# FIG 2